# EUROPÄISCHE PATENTANMELDUNG

(11) **EP 3 769 798 A1**
(43) Veröffentlichungstag der Anmeldung: **27.01.2021**
(21) Anmeldenummer: 19188341.2
(22) Anmeldetag: 25.07.2019
(51) Int. Cl.: A61M 1/00, A61M 1/06

(54) **MOTORBETRIEBENE MEDIZINISCHE SAUGPUMPE**

(71) Anmelder: Medela Holding AG, 6340 Baar (CH)
(72) Erfinder: Walter, Andreas, 8910 Affoltern am Albis (CH); Marbet, Regina, 4933 Rütschelen (CH)
(74) Vertreter: Grünecker Patent- und Rechtsanwälte PartG mbB

(57) **Zusammenfassung**

Die vorliegende Erfindung betrifft eine motorbetriebene medizinische Saugpumpe mit einem Pumpaggregat (2) zur Erzeugung eines Unterdrucks mit einem Gehäuse (40, 42, 44), das das Pumpaggregat (2) in sich aufnimmt, wobei das Pumpaggregat (2) eine Vakuumöffnung (8) für den von dem Pumpaggregat (2) zu erzeugenden Unterdruck und zumindest eine weitere, mit der Vakuumöffnung (8) durch das Pumpaggregat (2) kommunizierende Öffnung (10) aufweist. Die vorliegende Erfindung gibt eine einfach herzustellende, kompakt bauende und mit guten Schalldämpfungseigenschaften versehene Saugpumpe der eingangs genannten Art an, die hierzu eine Schalldämpfungskammer (14) aufweist, die dicht an die weitere Öffnung angeschlossen ist und diese umschließt.

## Beschreibung

Die vorliegende Erfindung betrifft eine motorbetriebene medizinische Saugpumpe mit einem Pumpaggregat zur Erzeugung eines Unterdrucks und mit einem Gehäuse, das das Pumpaggregat in sich aufnimmt. Das Pumpaggregat hat eine Vakuumöffnung für den von dem Pumpaggregat zu erzeugenden Unterdruck und zumindest eine weitere, mit der Vakuumöffnung durch das Pumpaggregat kommunizierende Öffnung.

Eine derartige motorbetriebene medizinische Saugpumpe ist aus CN 202 342 540 U1 bekannt. Bei diesem Stand der Technik ist die weitere Öffnung eine Entlüftungsöffnung, aus der bei dem zyklischen Betrieb des Pumpaggregats zur Erzeugung des Unterdrucks Luft aus dem Pumpaggregat ausgebracht wird. Die Erzeugung des Unterdrucks an der Vakuumöffnung bedingt das Austreiben von Luft aus dem Pumpaggregat. Hierzu weist dieses die besagte Entlüftungsöffnung auf.

Die auf die Anmelderin zurückgehenden Vorveröffentlichungen WO 2019/096700 A2 und WO 2006/032156 A1 offenbaren ein Pumpaggregat mit einer Vakuumöffnung, einer Auslassöffnung und einer Belüftungsöffnung, wobei die Belüftungsöffnung über ein Belüftungsventil mit einer Vakuum führenden Vakuumleitung innerhalb des Pumpaggregates verbunden werden kann, um einen übermäßigen Unterdruck abzubauen. Diese Reduktion des Unterdrucks innerhalb des Pumpaggregats ist dem Umstand geschuldet, dass die im Stand der Technik beschriebene motorbetriebene medizinische Saugpumpe eine Brustpumpe ist und dass beim Abpumpen von Muttermilch aus der weiblichen Brust ein gewisses Unterdruckniveau nicht unterschritten werden sollte, da das Saugen anderenfalls als schmerzhaft empfunden wird, ggf. auch Schäden der weiblichen Brust zu befürchten sind.

Gemäß WO 2019/096700 A2 ist das Pumpaggregat in einem Pumpaggregatgehäuse vollständig aufgenommen. Die Entlüftungsöffnung bzw. die Belüftungsöffnung liegen innerhalb dieses Pumpaggregatgehäuses, in dem Stand der Technik als Etui beschrieben, frei. Das Pumpaggregatgehäuse ist mehrteilig ausgebildet. Durch die Entlüftungsöffnung austretende Luft soll durch im Bereich der Fügefläche zwischen zwei Gehäuseteilen, die das Pumpaggregatgehäuse bilden, vorgesehene Schlitze entweichen. Durch das das Pumpaggregat vollständig einschließende Pumpaggregatgehäuse ist ein gewisser Schallschutz gegeben.

Nach CN 202 342 540 U1 ist die Entlüftungsöffnung über einen Entlüftungsschlauch mit einer Schalldämpfungskammer verbunden, in welcher ein schallabsorbierender Schaumstoff vorgesehen ist und die mit mehreren Auslassöffnungen versehen ist.

Der vorliegenden Erfindung liegt das Problem zugrunde, eine motorbetriebene medizinische Saugpumpe der eingangs genannten Art anzugeben, die sich einfach herstellen lässt, kompaktbauend ist und darüber hinaus eine wirkungsvolle Schalldämpfung bereitstellt.

Zur Lösung dieses Problems wird mit der vorliegenden Erfindung eine motorbetriebene medizinische Saugpumpe mit den Merkmalen von Anspruch 1 vorgeschlagen. Diese unterscheidet sich von der gattungsgemäßen motorbetriebenen medizinischen Saugpumpe dadurch, dass die Schalldämpfungskammer dicht an die weitere Öffnung angeschlossen ist und diese umgibt.

Die weitere Öffnung im Sinne der vorliegenden Erfindung kann die Entlüftungsöffnung sein. Sie kann aber auch die Belüftungsöffnung gemäß WO 2019/096700 A2 sein. Die Öffnung ist jedenfalls eine Öffnung, die über das Pumpaggregat, d.h. durch innerhalb des Pumpaggregates ausgebildete Strömungswege mit der Vakuumöffnung und innerhalb des Pumpaggregates kommuniziert. Die Entlüftungsöffnung kommuniziert üblicherweise über eine Pumpkammer, in der beispielsweise ein Kolben zyklisch bewegt wird, mit der Vakuumöffnung, wobei zwischen der Vakuumöffnung und der Entlüftungsöffnung zumindest ein stellbares Ventil vorgesehen sein kann. Die Belüftungsöffnung nach WO 2019/096700 A2 kommuniziert bei geöffnetem Belüftungsventil unmittelbar mit einer zu der Vakuumöffnung führenden Vakuumleitung innerhalb des Pumpaggregats. So sind sowohl die Belüftungsöffnung als auch die Entlüftungsöffnung weitere Öffnungen im Sinne der vorliegenden Erfindung.

Die weitere Öffnung im Sinne der vorliegenden Erfindung ist insbesondere die Entlüftungsöffnung des Pumpaggregats, aus der beim zyklischen Betrieb des Pumpaggregats zur Erzeugung des Unterdrucks Luft aus dem Pumpaggregat ausgebracht wird. Der hier aus dem Pumpaggregat austretende Schall ist üblicherweise stärker als der im Bereich der Belüftungsöffnung abgegebene Schall.

Die erfindungsgemäße Schalldämpfungskammer ist dicht an die weitere Öffnung angeschlossen und umschließt diese. Die Schalldämpfungskammer ist damit unmittelbar an die weitere Öffnung angeschlossen und nicht wie beim Stand der Technik gemäß CN 202 342 540 U1 über einen Schlauch. So erlaubt die erfindungsgemäße Lösung einen kompakten Aufbau.

Durch das unmittelbare Anschließen der weiteren Öffnung an die Schalldämpfungskammer tritt der von dem Pumpaggregat im Bereich der weiteren Öffnung abgehende Schall direkt in die Schalldämpfungskammer ein und wird dort wirkungsvoll gedämpft. Die Schalldämpfungskammer umschließt nach der vorliegenden Erfindung die weitere Öffnung, was bedeutet, dass sich die weitere Öffnung und damit ein gewisser Flächenanteil der äußeren Umhüllung des Pumpaggregates innerhalb der Schalldämpfungskammer befindet. Dieser Flächenanteil kann allein durch einen Auslassstutzen gebildet sein, der die weitere Öffnung, insbesondere die Entlüftungsöffnung umschließen und ausbilden kann. Die Schalldämpfungskammer kann aber auch noch weitere Flächenanteile des Pumpaggregatgehäuses in sich aufnehmen. Jedenfalls liegt die durch das Pumpaggregat gebildete weitere Öffnung, bevorzugt die Entlüftungsöffnung in der Schalldämpfungskammer und infolge des Umschließens der Öffnung durch diese Schalldämpfungskammer unmittelbar in der Schalldämpfungskammer frei.

Die weitere Öffnung ist dicht an die Schalldämpfungskammer angeschlossen. Dicht bedeutet hier schalldicht in dem Sinne, dass der durch die weitere Öffnung abgegebene Schall nicht ungehindert von der weiteren Öffnung nach außen gelangen kann, sondern zunächst in die Schalldämpfungskammer eingeleitet wird. Die Schalldämpfungskammer nimmt beispielsweise einen die weitere Öffnung umgebenden Stutzen des Pumpaggregatgehäuses abgedichtet in sich auf. Alternativ kann die Schalldämpfungskammer auch schalldicht an einen Flächenbereich des Pumpaggregatgehäuses angeschlossen sein, in dem die weitere Öffnung freiliegt.

Die Auslassöffnung der Schalldämpfungskammer liegt in der Regel in dem Gehäuse der Saugpumpe oder an der Außenseite dieses Gehäuses frei. Die Auslassöffnung ist in der Regel nicht an eine andere Kammer oder einen Schlauch unmittelbar angeschlossen.

Ein Pumpaggregat der erfindungsgemäßen Saugpumpe ist insbesondere ein Aggregat, welches einen Motor sowie eine Arbeitseinheit umfasst, die antriebstechnisch mit dem Motor verbunden ist und zumindest einen Arbeitsraum aufweist, in dem aufgrund der Antriebsleistung des Motors ein an der Vakuumöffnung wirksamer Unterdruck erzeugt werden kann. Eine solche Antriebseinheit kann beispielsweise einen über einen Exzenter mit der Motorwelle verbundenen und über eine in dem Pumpaggregatgehäuse abgedichtete Membran abgedichteter und beweglich gehaltener Kolben sein, wie er beispielsweise aus WO 2006/032156 A1 bekannt ist.

Das Gehäuse der motorbetriebenen medizinischen Saugpumpe kann das äußere Gehäuse der Saugpumpe sein, an dessen Außenseite Bedienelemente für das Steuern der Saugpumpe und/oder ein Vakuumanschluss für den Anschluss eines zu einer Brusthaube zum Anlegen an die Mutterbrust führenden Saugschlauchs und/oder ein Stecker zum Anschluss eines Leistungsstroms an das Pumpaggregat vorgesehen sein kann. Zwar ist die erfindungsgemäße motorbetriebene medizinische Saugpumpe insbesondere eine Saugpumpe wie sie in einem Brustpumpengerät zum Abpumpen von menschlicher Muttermilch üblicherweise zum Einsatz kommt. Die erfindungsgemäße Pumpe eignet sich jedoch auch für andere Anwendungen, beispielsweise als Drainagepumpe zum Absaugen von Körperflüssigkeiten.

In dem Gehäuse der erfindungsgemäßen Saugpumpe liegt üblicherweise die Auslassöffnung der Schalldämpfungskammer frei. So bewirkt das Gehäuse einen zusätzlichen Schalldämpfungseffekt gegenüber etwaigem Schall, der an der Auslassöffnung ggf. austritt. Das Gehäuse ist üblicherweise mehrteilig ausgebildet und hat üblicherweise Halterungen zur Aufnahme des Pumpaggregates bzw. eines Energiespeichers in Form einer Batterie oder eines Akkumulators. Der zuvor bereits erwähnte Vakuumanschluss liegt üblicherweise an der Außenseite des Gehäuses frei und ist dort zur Aufnahme eines Steckers eines Saugschlauches, z.B. der zu der Brusthaube führt, angepasst ausgebildet.

Wie zuvor bereits dargelegt, ist die Schalldämpfungskammer unmittelbar an das Pumpaggregat angeschlossen, bevorzugt mit dem Gehäuse des Pumpaggregates verbunden. Hierdurch ergibt sich eine einfache Herstellbarkeit der erfindungsgemäßen Saugpumpe. Denn die Schalldämpfungskammer muss lediglich mit dem Pumpaggregat verbunden werden und kann in dieser Weise einheitlich mit dem Pumpaggregat vorbereitet und im Rahmen der Montage gehandhabt werden.

Im Hinblick auf eine gute Schalldämpfung innerhalb der Schalldämpfungskammer wird gemäß einer bevorzugten Weiterbildung der vorliegenden Erfindung vorgeschlagen, die weitere Öffnung gegenüberliegend zu der Auslassöffnung vorzusehen. Dadurch wird ein größtmöglicher Abstand zwischen der weiteren Öffnung und der Auslassöffnung geschaffen, der sich günstig auf die Schalldämpfung innerhalb der Schalldämpfungskammer auswirkt. Die weitere Öffnung und die Auslassöffnung können dabei beispielsweise auf einer gemeinsamen Achse liegen, die sich parallel zu der Antriebsachse eines Motors des Pumpaggregates erstreckt. Eine achssymmetrische Anordnung ist dabei fertigungstechnisch besonders einfach zu verwirklichen und schafft regelmäßig einen relativ großen Abstand der Auslassöffnung auch zu benachbarten Wandungen des Gehäuses, welches wie oben beschrieben eine sekundäre Schalldämpfung bewirken kann.

Praktische Versuche haben ergeben, dass eine besonders gute Schalldämpfung mit einer Ausgestaltung bewirkt wird, bei welcher die weitere Öffnung einen größeren Strömungsquerschnitt als die Auslassöffnung hat. Das Durchmesserverhältnis zwischen dem Durchmesser der weiteren Öffnung, insbesondere der Entlüftungsöffnung und dem Durchmesser der Auslassöffnung sollte dabei zwischen 0,5 und 0,95 bevorzugt 0,7 liegen.

Bei den hier in Rede stehenden motorbetriebenen medizinischen Saugpumpen insbesondere für das Absaugen von Muttermilch aus der weiblichen Brust hat es sich als vorteilhaft erwiesen, die Schalldämpfungskammer mit einem Volumen zumindest 6 ml auszubilden. Unterhalb dieses Grenzwertes ergibt sich eine zunehmend unzureichende Schalldämpfung. Aus Gründen einer kompakten Bauweise hat es sich als vorteilhaft erwiesen, das Volumen der Schalldämpfungskammer auf 30 ml bevorzugt 29 ml zu beschränken. Diese Vorgaben gelten insbesondere für eine motorbetriebene medizinische Saugpumpe, die einen Durchsatz von zwischen 3 und 7 Liter/min hat und/oder auf der Saugseite und an der Vakuumöffnung einen Unterdruck von etwa 250 bis 300 mmHg erzeugen kann.

Gemäß einer bevorzugten Weiterbildung der vorliegenden Erfindung ist ein die Schalldämpfungskammer umgebendes Schalldämpfungsgehäuse steif ausgebildet. Das Schalldämpfungsgehäuse ist bevorzugt aus einem Kunststoff, besonders bevorzugt aus ABS ausgebildet. Aber auch andere Kunststoffe wie PA, PC, PP oder PS sind zur Ausbildung des Schalldämpfergehäuses denkbar.

Gemäß einer bevorzugten Weiterbildung der vorliegenden Erfindung ist die Schalldämpfungskammer durch ein Schlauchstück gebildet. Dieses Schlauchstück ist unmittelbar an das Pumpaggregatgehäuse angeschlossen, üblicherweise an einen Stutzen, der von dem Pumpaggregatgehäuse abragt. Bei dieser Ausgestaltung bildet das Schlauchstück selbst die Schalldämpfungskammer. So ist ein Ende des Schlauches unmittelbar mit der weiteren Öffnung verbunden und daran unmittelbar angeschlossen. Das andere Ende des Schlauches bildet die Auslassöffnung aus und liegt in dem Gehäuse frei. Diese Auslassöffnung kann durch das freie, abgeschnittene Ende des Schlauchstücks ausgebildet werden. Die Auslassöffnung kann aber auch durch einen Stopfen ausgeformt sein, der dicht in das freie Ende des Schlauchstücks eingesetzt ist, um eine Auslassöffnung mit einem von dem Lumen des Schlauches abweichenden Durchmesser bereitzustellen.

Eine besonders einfach herzustellende und kompakt bauende Ausgestaltung der vorliegenden Erfindung ist mit einer bevorzugten Ausgestaltung der vorliegenden Erfindung gegeben, bei welcher die Schalldämpfungskammer an dem Pumpaggregat befestigt ist. Das die Schalldämpfungskammer umgebende Schalldämpfungsgehäuse hat hierzu üblicherweise unmittelbar einteilig durch ein Kunststoffgehäuse ausgeformte Befestigungselemente, die mit dem Pumpaggregat, in der Regel dem Pumpaggregatgehäuse zusammenwirken. Die Schalldämpfungskammer kann mit dem Pumpaggregat verklebt, verschraubt oder verschweißt sein. Im Hinblick auf eine einfache Montage sind formschlüssige Verbindungselemente zu bevorzugen, die die Dämpfungskammer mit dem Pumpaggregat verbinden.

So wird gemäß einer bevorzugten Weiterbildung der vorliegenden Erfindung vorgeschlagen, dass ein die Schalldämpfungskammer umgebendes Schalldämpfungsgehäuse mit dem Pumpaggregat verrastet ist. Bei dieser Ausgestaltung wird üblicherweise das Schalldämpfungsgehäuse aus Kunststoff zusammen mit Rasthaken hergestellt, die das Pumpaggregat ganz oder teilweise umgreifen und damit verrasten. Die Ausgestaltung des Schalldämpfungsgehäuses aus Kunststoff hat dabei den Vorteil, dass die Rasthaken die für das Verrasten gewünschte Elastizität und das entsprechende Rückstellvermögen aufweisen.

Im Hinblick auf eine einfache dichte Anlage des Schalldämpfungsgehäuses an dem Pumpaggregat ist es zu bevorzugen, das Schalldämpfungsgehäuse mit einem Befestigungsflansch zu versehen, bevorzugt einteilig an dem Schalldämpfungsgehäuse einen solchen Befestigungsflansch anzuformen, der dicht an mindestens zwei sich im Wesentlichen rechtwinklig zueinander erstreckenden Flächenabschnitten des Pumpaggregats, speziell des Pumpaggregatgehäuses anliegt. Mit dieser Ausgestaltung werden zumindest zwei Flächenabschnitte einer Labyrinthdichtung zwischen dem Schalldämpfungsgehäuse und dem Pumpaggregat verwirklicht, die für eine gute schalldichte Anlage sorgen. Bevorzugt sind mehrere solcher sich jeweils rechtwinklig zueinander erstreckender Flächenabschnitte hintereinander angeordnet und liegen an einer entsprechend ausgebildeten Kontur des Befestigungsflansches an.

Das Pumpaggregat nach der vorliegenden Erfindung ist bevorzugt zumindest abschnittsweise zylindrisch ausgebildet. Ein Zylinderabschnitt wird üblicherweise durch den Motor des Pumpaggregates vorgegeben, der in der Regel ein Elektromotor ist. Bevorzugt schließt sich an das Motorgehäuse dieses Motors das Gehäuse der Arbeitseinheit an. Dieses Gehäuse der Arbeitseinheit kann in etwa mit gleichem Durchmesser wie das Motorgehäuse verwirklicht sein, wodurch ein abschnittsweise zylindrisches Pumpaggregat geschaffen wird, welches sich platzsparend in dem Gehäuse der erfindungsgemäßen Saugpumpe unterbringen lässt. Dabei hat üblicherweise das Gehäuse der Arbeitseinheit einen gegenüber dem Motorgehäuse vergrößerten Durchmesser, wodurch sich eine Stufe ergibt, gegen welche ein Rasthaken des Schalldämpfergehäuses verrastet werden kann, um dieses Schalldämpfergehäuse mit dem Pumpaggregat zu verbinden. Auch das Schalldämpfungsgehäuse ist bevorzugt zylindrisch ausgebildet, und zwar in etwa mit dem gleichen Durchmesser des Gehäuses der Arbeitseinheit und/oder des Motorgehäuses. So wird ein schallgedämpftes Pumpaggregat mit einer zumindest abschnittsweise zylindrischen Gestaltung geschaffen, die sich platzsparend in dem Gehäuse unterbringen lässt.

Gemäß einer weiteren bevorzugten Ausgestaltung der vorliegenden Erfindung ist das die Schalldämpfungskammer umgebende Schalldämpfungsgehäuse einerseits offen ausgebildet und durch das Pumpaggregat verschlossen. Diese Ausgestaltung bietet die Möglichkeit einer einfachen Herstellung des Schalldämpfungsgehäuses mittels Spritzgießen, denn das Schalldämpfungsgehäuse ist bei dieser Ausgestaltung als einseitig offener Topf ausgebildet, der sich beim Spritzgießen leicht entformen lässt. Dabei ragen von dem die Schalldämpfungskammer umfänglich und endseitig umgebenden, die eigentliche Schalldämpfungskammer ausbildenden Bereich des Schalldämpfungsgehäuses bevorzugt Rasthaken ab, die einteilig an dem Spritzgießteil angeformt sind und mit dem Pumpaggregat zur Befestigung des Schalldämpfungsgehäuses an dem Pumpaggregat zusammenwirken. Der dichte Anschluss eines solchen Schalldämpfungsgehäuses erfolgt bevorzugt über den zuvor bereits diskutierten Befestigungsflansch, der mit den äußeren Konturen des Pumpaggregats eine Labyrinthdichtung ausformt.

Dem gegenüber bietet eine alternative Ausgestaltung, bei der das Schalldämpfungsgehäuse die Schalldämpfungskammer vollumfänglich umgibt und lediglich über die weitere Öffnung und die Auslassöffnung nach außen offen ist, den Vorteil einer maximal geschlossenen und daher hinsichtlich der Schalldämpfung optimal ausbildbaren Schalldämpfungskammer. Dabei ist die Auslassöffnung üblicherweise als einfache Bohrung in einer Wandung der Schalldämpfungskammer ausgespart. Die weitere Öffnung befindet sich bevorzugt innerhalb eines von dem Pumpaggregat abragenden Stutzens, der von dem Schalldämpfungsgehäuse umgriffen ist, sodass die an dem freien Ende des Stutzens vorgesehene Entlüftungsöffnung sich innerhalb der Schalldämpfungskammer befindet und das Schalldämpfungsgehäuse den Stutzen dicht umschließt, bevorzugt von dem Stutzen gehalten ist. Dazu ist eine dem Stutzen zugeordnete Bohrung der Schalldämpfungskammer so auf die Außenumfangsfläche des Stutzens angepasst, dass sich eine dichte Verpressung zwischen dem Stutzen und dem Schalldämpfungsgehäuse ergibt. Das Schalldämpfungsgehäuse kann mit dem Stutzen kraft-, form- oder stoffschlüssig verbunden sein, insbesondere verklebt sein, sodass eine dauerhafte Verbindung zwischen dem Schalldämpfungsgehäuse und dem Pumpaggregat gegeben ist.

Im Hinblick auf eine kompakte Anordnung der wesentlichen Komponenten der medizinischen Saugpumpe wird gemäß einer bevorzugten Weiterbildung der vorliegenden Erfindung vorgeschlagen, das Pumpaggregat auf einem Pumpaggregatträger zu montieren. Dieser Pumpaggregatträger hat eine Aufnahme für einen Energiespeicher. Der Energiespeicher ist dabei bevorzugt ein längliches Bauteil, welches parallel zu dem zumindest abschnittsweise zylindrischen Pumpaggregat mit Schalldämpfungsgehäuse vorgesehen ist. So können die beiden Teile in paralleler Anordnung relativ zueinander auf dem Pumpaggregatträger montiert sein. Der Energiespeicher und das Pumpaggregat zusammen mit dem Schalldämpfungsgehäuse sind dabei so aufeinander abgestimmt, dass diese in etwa die gleiche axiale Länge haben. So geht von dem Pumpaggregat auf Höhe des Energiespeichers seitlich ein Vakuumschlauch ab, der mit der Vakuumöffnung verbunden ist und diese mit einer Durchgangsöffnung verbindet, die üblicherweise an dem Gehäuse außen freiliegt und mit dem zuvor bereits erwähnten Vakuumanschluss versehen ist, um dort einen Stecker eines Saugschlauches, der beispielsweise zu der Brusthaube führt, strömungsmäßig und mechanisch mit der Saugpumpe zu verbinden.

An dem Vakuumschlauch ist bei dieser Ausgestaltung bevorzugt eine gesteuert stellbare Belüftungsöffnung angeschlossen, die ein übermäßiges Unterdruckniveau im Bereich der Vakuumleitung und damit im Bereich des Vakuumanschlusses vermeiden soll. Details der Funktion einer solchen stell- bzw. steuerbaren Belüftungsöffnung sind in WO 2006/032156 A1 beschrieben.

Für die stellbare Belüftungsöffnung ist üblicherweise ein Belüftungsventil vorgesehen, welches in axialer Verlängerung des Energiespeichers bzw. des Pumpaggregats vorgesehen ist. Denn die parallele Anordnung von Pumpaggregat und Energiespeicher erfolgt mit dem Ziel, diese platzgreifenden Komponenten der Saugpumpe im Wesentlichen über die gesamte Breitenerstreckung des Pumpaggregatträgers anzuordnen. So bleibt bei kompakter Anordnung der Komponenten in axialer Verlängerung des Energiespeichers bzw. Pumpaggregats Platz für die Aufnahme weiterer Komponenten.

Aus gleichen Überlegungen wird gemäß einer bevorzugten Ausgestaltung der vorliegenden Erfindung vorgeschlagen, das Pumpaggregat durch einen länglichen Körper auszubilden, der an seinem einen Ende die Schalldämpfungskammer und an seinem anderen Ende einen Motor des Pumpaggregates aufweist und diese Einheit in im Wesentlichen paralleler Anordnung zu dem Energiespeicher auf dem Pumpaggregatträger zu montieren, der das zuvor erwähnte Belüftungsventil dem Energiespeicher vorgelagert trägt.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
- Figur 1: eine Längsschnittansicht eines Pumpaggregats mit einem ersten Ausführungsbeispiel einer Schalldämpfungskammer;
- Figur 2: eine Längsschnittansicht eines Pumpaggregats mit einem zweiten Ausführungsbeispiel einer Schalldämpfungskammer;
- Figur 3: eine Längsschnittansicht eines Pumpaggregats mit einem dritten Ausführungsbeispiel einer Schalldämpfungskammer;
- Figur 4: eine Explosionsdarstellung eines Ausführungsbeispiels einer motorbetriebenen medizinischen Saugpumpe;
- Figur 5: eine Draufsicht auf das in Figur 4 dargestellte Ausführungsbeispiel und
- Figur 6: eine perspektivische Seitenansicht des Ausführungsbeispiels nach den Figuren 4 und 5.

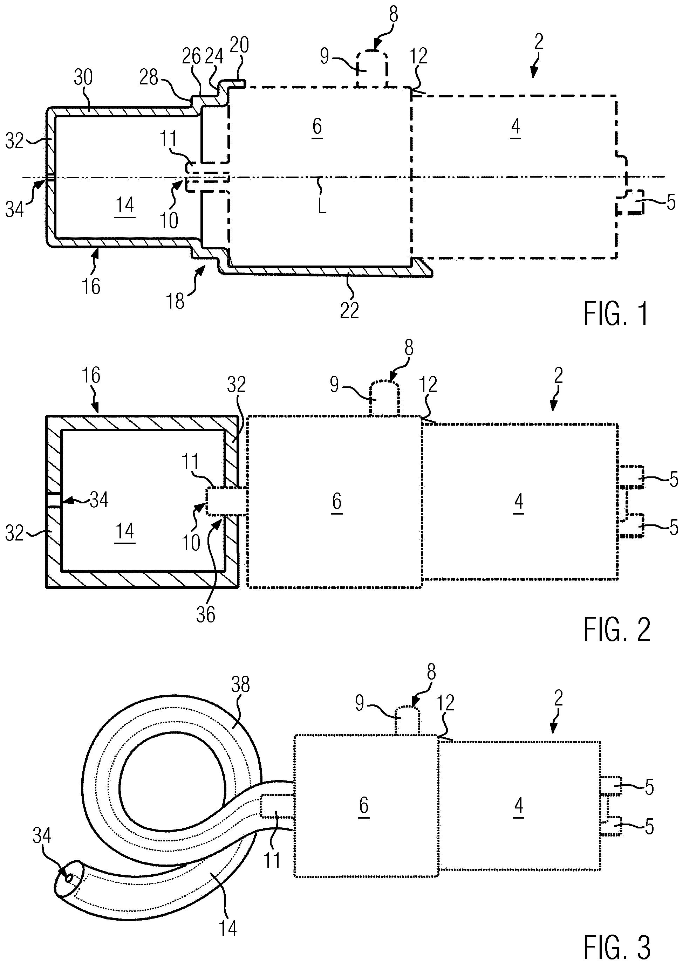

In den Figuren 1 bis 3 ist mit Bezugszeichen 2 ein Pumpaggregat gekennzeichnet, welches einen Elektromotor 4 und eine Arbeitseinheit 6 aufweist. Die Arbeitseinheit 6 kann die in WO 2006/032156 A1 beschriebenen Komponenten aufweisen und in ähnlicher Weise aufgebaut sein. Der Elektromotor 4 ist zylindrisch ausgebildet, wobei von der freien Stirnseite elektrische Anschlusszungen 5 abragen. An der gegenüberliegenden Stirnseite schließt die ebenfalls im Wesentlichen zylindrisch ausgebildete Arbeitseinheit 6 an, von der in Umfangsrichtung ein eine Vakuumöffnung 8 umschließender Vakuumstutzen 9 abragt und an der dem Elektromotor 4 gegenüberliegend eine Entlüftungsöffnung 10 vorgesehen ist, die von einem Entlüftungsstutzen 11 gebildet ist, der dort von einer Stirnfläche der Arbeitseinheit 6 abragt. Der Durchmesser des Elektromotors 4 ist etwas kleiner als der Durchmesser der Arbeitseinheit 6. Dadurch wird zwischen den beiden Komponenten 4, 6 eine Ringfläche 12 gebildet.

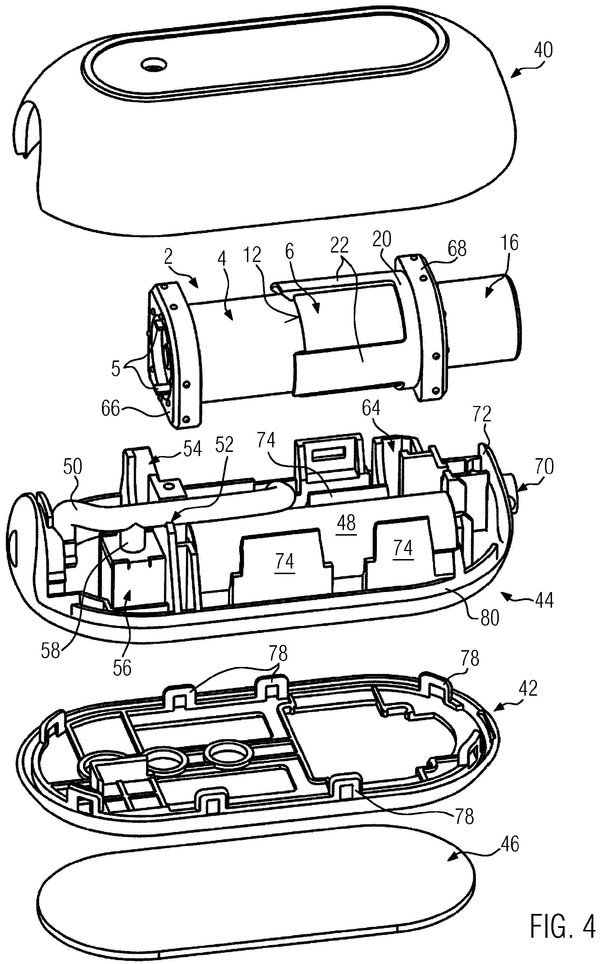

Die Figur 1 zeigt ein erstes Beispiel einer Schalldämpfungskammer 14, die vorliegend durch ein topfförmiges, einseitig offenes Schalldämpfungsgehäuse 16 gebildet ist, welches gegenüberliegend zu der die Entlüftungsöffnung 10 aufweisenden Stirnfläche von der Arbeitseinheit 6 abgeschlossen wird. Das Schalldämpfungsgehäuse 16 hat einen Befestigungsflansch 18 mit einem mehrfach gestuften Verlauf. Der Befestigungsflansch 18 hat einen äußeren Ringabschnitt 20, der die Außenumfangsfläche der Arbeitseinheit 6 endseitig umgreift und von dem Rasthaken 22 abragen, die gegen die Ringfläche 12 verriegeln. Wie insbesondere die Figuren 4 bis 6 verdeutlichen, gehen von dem äußeren Ringabschnitt 22 mehrere, in Umfangsrichtung verteilt vorgesehene Rasthaken 22 ab und sind in der zuvor erwähnten und in Figur 1 zu erkennenden Weise mit dem Pumpaggregat 2 formschlüssig verbunden. Der äußere Ringabschnitt 20 ist über einen äußeren Radialabschnitt 24, einen inneren Ringabschnitt 26 und einen inneren Radialabschnitt 28 mit einem Zylinderabschnitt 30 verbunden, der die eigentliche Schalldämpfungskammer 14 umfänglich umgibt und mit einer stirnseitigen Kappe 32 versehen ist, die eine Auslassöffnung 34 aufweist. Der Befestigungsflansch 18 mit dem zuvor beschriebenen gestuften Aufbau, der Zylinderabschnitt 30 und die Kappe 32 sind durch ein einheitliches Kunststoffbauteil ausgeformt, welches auch die Rasthaken 22 ausbildet. Das Kunststoffbauteil ist vorliegend aus ABS spritzgegossen.

In Figur 1 ist das Gehäuse der Arbeitseinheit 6 strichpunktiert dargestellt. Bei dieser Ausformung der Arbeitseinheit 6 liegt lediglich der äußere Ringabschnitt 20 und der äußere Radialabschnitt 24 an Flächenabschnitten der Arbeitseinheit 6 an, die sich rechtwinklig zueinander erstrecken. Allerdings kann die Arbeitseinheit 6 auch einen gestuften Verlauf aufweisen, der dem gestuften Verlauf des Befestigungsflansches 18 entspricht, sodass sämtliche Flächenabschnitte des Befestigungsflansches 18, die von dem inneren Radialabschnitt 28 bis zu dem äußeren Ringabschnitt 20 gebildet werden, an der Arbeitseinheit 6 anliegen, wodurch eine sehr wirksame Labyrinthdichtung gebildet ist, über welche das Schalldämpfungsgehäuse 16 dicht an der Arbeitseinheit 6 anliegt. So ist die Schalldämpfungskammer 14 im Wesentlichen luftdicht mit dem Pumpaggregat 2 verbunden.

Wie Figur 1 verdeutlicht, hat die Auslassöffnung 34 einen geringeren Durchmesser als die Entlüftungsöffnung 10. Sie liegt zusammen mit der Entlüftungsöffnung 10 auf einer durch die Welle des Elektromotors 2 vorgegebenen Achse L.

Entsprechende Durchmesserverhältnisse sind auch bei dem zweiten Beispiel einer Schalldämpfungskammer nach Figur 2 verwirklicht. Gleiche Bauteile sind mit gleichen Bezugszeichen gekennzeichnet. Die Schalldämpfungskammer 14 wird durch ein Schalldämpfungsgehäuse 16 umgeben, welches zylindrisch ausgebildet und in Umfangsrichtung vollends geschlossen und lediglich an seinen gegenüberliegenden, sich quer zu einer Axialrichtung des zylindrischen Körpers erstreckenden Kappen 32 einerseits die Auslassöffnung 34 und andererseits eine Aufnahmebohrung 36 aufweist, die an den Außendurchmesser des Entlüftungsstutzens 11 angepasst ausgebildet ist, sodass das Schalldämpfungsgehäuse 16 gegenüber diesem Entlüftungsstutzen 11 abgedichtet und an diesem gehalten ist. Das Schalldämpfungsgehäuse 16 ist mit dem Entlüftungsstutzen 11 verklebt oder in anderer Weise luftdicht verbunden ist.

Eine entsprechende Befestigung der Schalldämpfungskammer 14 an dem Pumpaggregat 2 verdeutlicht auch Figur 3. Dort ist die Schalldämpfungskammer 14 durch das Lumen eines Schlauches 38 gebildet, der auf den Entlüftungsstutzen 11 dicht aufgeschoben ist und aus einem weichelastischen Kunststoff, beispielsweise Silikon, bestehen kann. Das freie Ende des Schlauches 38 bildet die Auslassöffnung 34 aus. Auch diese Auslassöffnung 34 hat bevorzugt einen kleineren Durchmesser als die Entlüftungsöffnung 10 und auch einen gegenüber dem Durchmesser der Schalldämpfungskammer 14 verminderten Durchmesser. Dieser verminderte Durchmesser kann durch einen Stopfen verwirklicht werden, der mit dem Schlauch 38 verbunden ist. Die Auslassöffnung ist eine freie Öffnung, die in einem Gehäuse der Pumpe freiliegt.

Die Figuren 4 bis 6 zeigen ein Ausführungsbeispiel einer motorbetriebenen medizinischen Saugpumpe unter Verwendung eines Pumpaggregats 2 mit Schalldämpfungsgehäuse 16 entsprechend dem ersten Ausführungsbeispiel nach Figur 1. Gleiche Bauteile sind hier auch mit gleichen Bezugszeichen gekennzeichnet.

Das Ausführungsbeispiel weist einen Gehäusedeckel 40 und einen Gehäuseboden 42 auf, die in geschlossenem Zustand einen Pumpaggregatträger 44 zwischen sich aufnehmen. Zwischen dem Pumpaggregatträger 44 und dem Gehäuseboden 42 kann eine nicht gezeigte bestückte Leiterplatte mit Steuerelementen vorgesehen sein, deren Bedienelemente gegenüber einer mit Bezugszeichen 46 gekennzeichneten Abdeckung freiliegen, wie dies in WO 2019/096700 A2 beschrieben ist. Die Elemente 40 bis 44 bilden ein Gehäuse des Ausführungsbeispiels aus.

Diese Offenlegungsschrift vermittelt weitere Informationen zu Details des Ausführungsbeispiels, die auch vorliegend verwirklicht sind. So kann auf die frühere Offenbarung verwiesen werden.

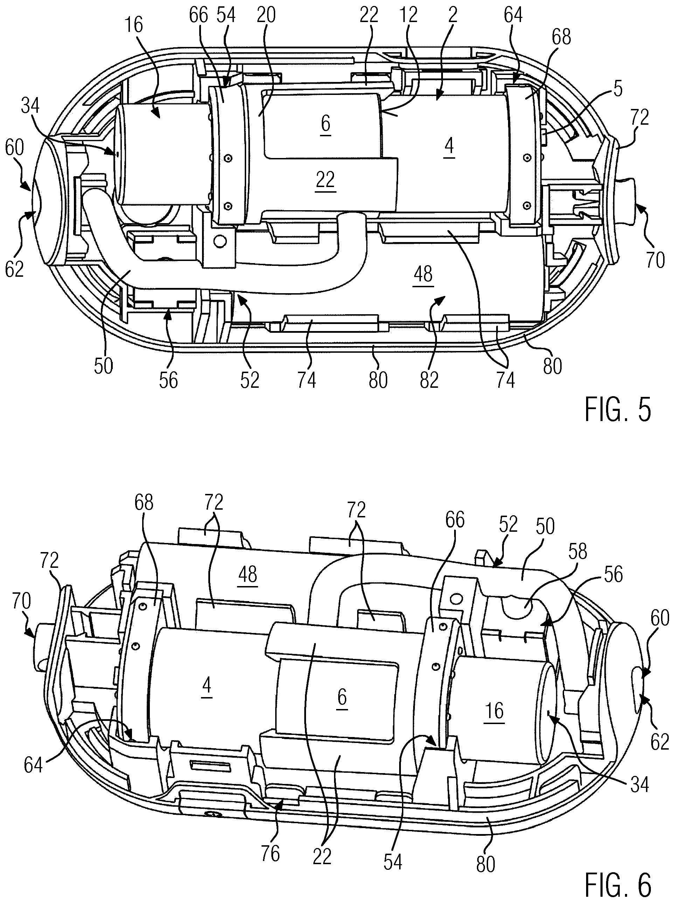

Abweichend von der Offenbarung der WO 2019/096700 A2 sind auf dem Pumpaggregatträger 44 vorliegend ein Energiespeicher 48 und das Pumpaggregat 2 in paralleler Anordnung vorgesehen. Dabei hat das Pumpaggregat 2 (ohne die Schalldämpfungskammer 14) in etwa die gleiche axiale Länge wie der Energiespeicher 48 (vgl. Figur 5). Dort ist auch ersichtlich, dass ein mit Bezugszeichen 50 gekennzeichneter und an dem Vakuumstutzen 9 angeschlossener Vakuumschlauch in etwa auf mittlerer Höhe des Energiespeichers 48 zunächst radial von dem Pumpaggregat 2 abgeht und danach in axialer Richtung des Energiespeichers 48 und oberhalb desselben sowie parallel dazu hinausgeführt wird. Der Vakuumschlauch 50 ist in einer Schlauchaufnahme 52 geklemmt, die durch eine den Pumpaggregatträger 44 in Breitenrichtung durchsetzende Wand gebildet ist, die in Verlängerung der Schlauchaufnahme 52 eine erste Pumpaggregataufnahme 54 ausformt. Unmittelbar benachbart zu der Schlauchaufnahme 52 befindet sich ein Belüftungsventil 56, das strömungsmäßig über eine Belüftungsleitung 58 mit dem Vakuumschlauch 50 verbunden ist. Die Funktion des Belüftungsventils ist insbesondere in WO 2006/032156 A1 beschrieben. Das Belüftungsventil 58 verhindert einen übermäßigen Unterdruck in dem Vakuumschlauch 50.

Das freie Ende des Vakuumschlauches 50 ist mit einem Vakuumanschluss 60 verbunden, der in einer Durchgangsöffnung 62 freiliegt, die durch den Pumpaggregatträger 44 gebildet ist. Die entsprechende Ausgestaltung dient als Aufnahme eines Steckers eines Saugschlauches, der zu der Brusthaube führt. Zur Abdichtung der Steckverbindung kann beispielsweise der Vakuumanschluss 60 aus einem weichelastischen Kunststoffmaterial, wie beispielsweise TPE oder Silikon gebildet sei.

Von dem Boden des Pumpaggregatträgers 44 ragt eine zweite Pumpaggregataufnahme 64 ab. Die beiden Pumpaggregataufnahmen 54, 64 befinden sich etwa höhengleich mit den axialen Enden des Energiespeichers 48. Sie nehmen erste und zweite Lagermodule 66, 68 auf, die vor der Montage des Pumpaggregats 2 auf dieses aufgeschoben worden sind. Das erste Lagermodul 66 hat dazu eine gestufte Bohrung, die im Inneren des ersten Lagermoduls 66 eine Ringfläche zur stirnseitigen Anlage des Elektromotors 4 ausformt. Eine entsprechend gestufte Bohrung ist auch bei dem zweiten Lagermodul 68 verwirklicht. Diese gestufte Bohrung bildet Anlageflächen für den inneren Radialabschnitt 28 und den inneren Ringabschnitt 26 aus. Nach Einbringen in die als Schiebeführung ausgebildeten Pumpaggregataufnahmen 54, 64 ist dementsprechend das Pumpaggregat 2 zusammen mit dem Schalldämpfungsgehäuse 16 fest auf dem Pumpaggregatträger 44 montiert.

Wie die Figuren 5 und 6 verdeutlichen, befindet sich das Belüftungsventil 56 auf Höhe der Schalldämpfungskammer 14. Die Schalldämpfungskammer 14 hat einen etwas geringeren Durchmesser als die Arbeitseinheit 6, sodass radial neben der Schalldämpfungskammer 14 hinreichend Platz für die Anordnung des Belüftungsventils 56 verbleibt.

An der gegenüberliegenden Stirnseite des Pumpaggregatträgers 44 ragt eine Kabeldurchführung 70 von einer Stirnwand 72 ab, die zwischen sich und den axialen Enden des Pumpaggregats 2 bzw. des Energiespeichers 48 hinreichend Platz für die Aufnahme von Kabeln erlaubt, die von der Kabeldurchführung 70 zu der nicht gezeigten Leiterplatte und von dort zu den Anschlusszungen 5 des Elektromotors 4 führen.

Insgesamt verdeutlicht das Ausführungsbeispiel eine kompakte Anordnung der Komponenten der Saugpumpe. Der Pumpaggregatträger 44 ist als Spritzgussteil ausgebildet und hat neben den Aufnahmen 52, 54, 64 für das Pumpaggregat 2 und den Vakuumschlauch 50 einteilig an dem Pumpaggregatträger 44 ausgeformte Klammern 74 zur Befestigung des Energiespeichers 48 und Rastkanten 76, die zur Befestigung mit dem Gehäuseboden 42 mit den dortigen Rastvorsprüngen 78 zusammenwirken, sowie einen aufstehenden Randabschnitt 80, mit dem der Gehäusedeckel 40 verbunden wird. Zwischen den Klammern 74 wird eine Aufnahme 82 für den Energiespeicher 48 gebildet.

### Bezugszeichenliste

- 2: Pumpaggregat
- 4: Elektromotor
- 5: Anschlusszunge
- 6: Arbeitseinheit
- 8: Vakuumöffnung
- 9: Vakuumstutzen
- 10: Entlüftungsöffnung
- 11: Entlüftungsstutzen
- 12: Ringfläche
- 14: Schalldämpfungskammer
- 16: Schalldämpfungsgehäuse
- 18: Befestigungsflansch
- 20: äußerer Ringabschnitt
- 22: Rasthaken
- 24: äußerer Radialabschnitt
- 26: innerer Ringabschnitt
- 28: innerer Radialabschnitt
- 30: Zylinderabschnitt
- 32: Kappe
- 34: Auslassöffnung
- 36: Aufnahmebohrung
- 38: Schlauch
- 40: Gehäusedeckel
- 42: Gehäuseboden
- 44: Pumpaggregatträger
- 46: Abdeckung
- 48: Energiespeicher
- 50: Vakuumschlauch
- 52: Schlauchaufnahme
- 54: erste Pumpaggregataufnahme
- 56: Belüftungsventil
- 58: Belüftungsleitung
- 60: Vakuumanschluss
- 62: Durchgangsöffnung
- 64: zweite Pumpaggregataufnahme
- 66: erstes Lagermodul
- 68: zweites Lagermodul
- 70: Kabeldurchführung
- 72: Stirnwand
- 74: Klammer
- 76: Rastkanten
- 78: Rastvorsprung
- 80: Randabschnitt
- 82: Aufnahme für den Energiespeicher

## Patentansprüche

1. Motorbetriebene medizinische Saugpumpe mit einem Pumpaggregat (2) zur Erzeugung eines Unterdrucks mit einem Gehäuse (40, 42, 44), das das Pumpaggregat (2) in sich aufnimmt, wobei das Pumpaggregat (2) eine Vakuumöffnung (8) für den von dem Pumpaggregat (2) zu erzeugenden Unterdruck und zumindest eine weitere, mit der Vakuumöffnung (8) durch das Pumpaggregat (2) kommunizierende Öffnung (10) aufweist, die mit einer eine Auslassöffnung (34) aufweisenden Schalldämpfungskammer (14) verbunden ist,
**dadurch gekennzeichnet,**
**dass** die Schalldämpfungskammer (40) dicht an die weitere Öffnung (10) angeschlossen ist und diese umschließt.

2. Motorbetriebene medizinische Saugpumpe nach Anspruch 1, **dadurch gekennzeichnet, dass** die weitere Öffnung eine Entlüftungsöffnung (10) des Pumpaggregats (2) ist.

3. Motorbetriebene medizinische Saugpumpe nach Anspruch 1 oder 2, **dadurch gekennzeichnet, dass** die weitere Öffnung (10) gegenüberliegend zu der Auslassöffnung (34) vorgesehen ist.

4. Motorbetriebene medizinische Saugpumpe nach einem der vorherigen Ansprüche, **dadurch gekennzeichnet, dass** die weitere Öffnung (10) einen größeren Strömungsquerschnitt als die Auslassöffnung (34) hat.

5. Motorbetriebene medizinische Saugpumpe nach einem der vorherigen Ansprüche, **dadurch gekennzeichnet, dass** die Schalldämpfungskammer (14) ein Volumen von mindestens 6 ml, bevorzugt nicht mehr als 30 ml hat.

6. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) steif, bevorzugt aus einem Kunststoff, besonders bevorzugt aus ABS ausgebildet ist.

7. Motorbetriebene medizinische Saugpumpe nach einem der vorherigen Ansprüche, **dadurch gekennzeichnet, dass** die Schalldämpfungskammer (14) durch ein Schlauchstück (38) gebildet ist.

8. Motorbetriebene medizinische Saugpumpe nach einem der vorherigen Ansprüche, **dadurch gekennzeichnet, dass** die Schalldämpfungskammer (14) an dem Pumpaggregat (2) befestigt ist.

9. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) mit dem Pumpaggregat (2) verrastet ist.

10. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) einen Befestigungsflansch (18) aufweist, der dicht an mindestens zwei, sich im Wesentlichen rechtwinklig zueinander erstreckenden Flächenabschnitten des Pumpaggregats (2) anliegt.

11. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) zylindrisch ausgebildet ist.

12. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) einseitig offen ausgebildet und durch das Pumpaggregat (2) verschlossen ist.

13. Motorbetriebene medizinische Saugpumpe nach einem der Ansprüche 1 bis 11, **dadurch gekennzeichnet, dass** ein die Schalldämpfungskammer (14) umgebendes Schalldämpfungsgehäuse (16) die Schalldämpfungskammer (14) vollumfänglich umgibt und lediglich über die weitere Öffnung (10) und die Auslassöffnung (34) nach außen offen ist.

14. Motorbetriebene medizinische Saugpumpe nach einem der vorherigen Ansprüche, **dadurch gekennzeichnet, dass** das Pumpaggregat (2) auf einem Pumpaggregatträger (44) montiert ist, der eine Aufnahme (82) für einen Energiespeicher (48) ausbildet, auf dessen Höhe seitlich von dem Pumpaggregat (2) ein Vakuumschlauch (50) abgeht, der die Vakuumöffnung (8) mit einer Durchgangsöffnung (62) verbindet und an den eine gesteuert stellbare Belüftungsöffnung angeschlossen ist.

15. Motorbetriebene medizinische Saugpumpe nach einem der vorherigen Ansprüche, **dadurch gekennzeichnet, dass** das Pumpaggregat (2) durch einen länglichen Körper gebildet ist, der an seinem einen Ende die Schalldämpfungskammer (14) und an seinem anderen Ende einen Motor (4) des Pumpaggregats (2) aufweist, und dass ein sich im Wesentlichen parallel zu dem Pumpaggregat (2) erstreckender länglicher Energiespeicher (48) zusammen mit dem Pumpaggregat (2) auf einem Pumpaggregatträger (44) montiert ist, der dem Energiespeicher (48) vorgelagert ein eine Belüftungsöffnung stellendes Belüftungsventil (56) trägt.
